# EUROPÄISCHE PATENTSCHRIFT

(11) **EP 0 461 290 B1**
(45) Veröffentlichungstag und Bekanntmachung des Hinweises auf die Patenterteilung: **12.01.1994**
(21) Anmeldenummer: 90111258.1
(22) Anmeldetag: 14.06.1990
(51) Int. Cl.: A61K 31/565, A61K 9/48, A61K 9/66

(54) **Flüssig befüllte Steckkapselpräparate**
Capsules with a liquid content
Capsules à couleur liquide

(43) Veröffentlichungstag der Anmeldung: 18.12.1991
(73) Patentinhaber: HENNING BERLIN GmbH CHEMIE- UND PHARMAWERK, D-12099 Berlin (DE)
(72) Erfinder: Lahr, Wolfgang, D-1000 Berlin 41 (DE)
(74) Vertreter: Werner, Hans-Karsten, Dr.Dipl.-Chem.

(56) Entgegenhaltungen:
- FR-A- 2 312 256
- PHARM. IND., Band 40, Nr. 6, 1978, Seiten 654-657; A. CUINE et al.: "Das Einbringen viskoser Lösungen von Aktivstoffen in Hartgelatinekapseln"

## Beschreibung

Die Erfindung betrifft flüssig befüllte Steckkapseln zur oralen Verabreichung enthaltend Oestradiol bzw. Oestradiolvalerat und/oder Medroxyprogesteronacetat.

Oestradiol bzw. Oestradiolvalerat werden sowohl zur postmenopausalen Hormonsubstitutionstherapie als auch zur prophylaktischen Verhinderung der Osteoporose verwendet, wobei das Östrogen auch mit einer Gestagenkomponente, vorzugsweise mit Medroxyprogesteronacetat, kombiniert werden kann. Oestradiol, wie auch seine Salze, insbesondere Oestradiolvalerat, sind praktisch wasserunlöslich (Martindale, The Extra Pharmacopoeia, 1982, Seiten 1425 bis 1428). Gleiches gilt für Medroxyprogesteronacetat (ibid., Seiten 1416 bis 1417). Aus diesem Grunde ist die perorale Verabreichung der kristallinen Substanzen in Form fester Darreichungsformen problematisch, da die Löslichkeit bzw. Lösungsgeschwindigkeit der Wirkstoffe der geschwindigkeitsbestimmende Schritt für die Resorption ist, welche wiederum die biologische Verfügbarkeit und damit den Therapieerfolg bestimmt.

Nach allgemeiner Auffassung sind in Wasser schwer- oder unlösliche kristalline Substanzen schlecht bioverfügbar, weshalb man derartige Wirkstoffe, die oral zugeführt werden, mikronisiert, um durch Vergrößerung der Oberfläche die Lösungsgeschwindigkeit zu erhöhen und damit die Bioverfügbarkeit zu verbessern. E. J. Anthal, et al. in Int. of Pharmaceutics 54 (1989), Seiten 33 bis 39, zeigen für Medroxyprogesteronacetat anhand von Blutspiegel-/Zeitkurven, daß das Ausmaß der Bioverfügbarkeit erheblich von der Korngröße des Wirkstoffes bei oraler Gabe abhängig ist.

Die Zerkleinerung bzw. Mikronisierung von Stoffen ist technisch sehr aufwendig, insbesondere dann, wenn Partikel < 10 µm reproduzierbar hergestellt werden sollen. Darüber hinaus ist die Herstellung fester Arzneiformen, wie Tabletten, in denen der Wirkstoff niedrig dosiert ist, schwierig, um eine gute Gleichverteilung des Wirkstoffes pro Darreichungsform zu erzielen. Feuchtgranulierungen zur Verbesserung der Gleichförmigkeit und Gleichverteilung des Wirkstoffes scheiden in solchen Fällen aus, da die Gefahr unkontrollierten Kristallwachstums besteht.

Die DE-OS 27 58 549 beschreibt ein Arzneimittel in Kapseln auf Basis von Oestradiolvalerat zur Behandlung von Depressionen. Dabei wird das Oestradiolvalerat mit üblichen Trägersubstanzen, Verdünnungsmitteln und Geschmackskorrigentien verarbeitet. Für Injektionen kommen insbesondere ölige Lösungen, wie z.B. Lösungen in Sesam-, Rizinus- und Baumwollsamenöl in Frage. Die Lösungen können auch oral appliziert werden.

Die DE-OS 30 07 251 beschreibt eine oral in Kapseln applizierbare Arzneiform mit einem Alkylsulfonsäureester des Äthinyloestradiols mit einem lipophilem Lösungsmittel aus aliphatischen Karbonsäureestern mit 12 bis 25 Kohlenstoffatomen. Als Co-Solvens wird Benzylalkohol verwendet. Als lipophile Lösungsmittel können auch Gemische von Ölen pflanzlicher oder tierischer Herkunft oder von Mono-, Di- oder Triglyceriden mit aliphatischen Karbonsäureestern mit 12 bis 25 C-Atomen verwendet werden.

Die Publikation von A. Cuine et al. in Pharm. Ind. 40, Nr. 6 (1978) Seiten 654 bis 657 beschäftigt sich mit dem Einbringen viskoser Lösungen von Aktivstoffen in Hartgelatinekapseln, indem dem Aktivstoff ein nicht oxidierbares Fett beigemischt und die Mischung in flüssigem Zustand in Hartgelatinekapseln gefüllt wird. Die Mischungen erstarren in der Hartgelatinekapsel.

Der bisherige Stand der Technik verwendet überwiegend Oestradiol bzw. Oestradiolvalerate in Weichgelatinekapseln. Die Herstellung von oestradiolhaltigen Hartgelatinekapseln ist lediglich in FR-A 2 312 256 beschrieben. Dort werden hartschalige Gelatinekapseln enthaltend Oestradiol-17β-dodecanoat, Chlormadinon-acetat und Laurinsäure beschrieben. Laurinsäure ermöglicht die Herstellung eines bei Raumtemperatur festen Gemisches, welches in die Hartgelatinekapsel abgefüllt wird. Da Laurinsäure in Wasser unlöslich ist, ist diese Zubereitung jedoch nicht in der Lage, den Wirkstoff rasch zur Verfügung zu stellen.

Aufgabe der Erfindung ist es daher, Sexualhormone der vorgenannten Art zu festen Darreichungsformen für die orale Verabreichung in Hartgelatinekapseln zur Verfügung zu stellen, die den Wirkstoff mit guter Gleichverteilung und Bioverfügbarkeit, vorzugsweise in gelöstem Zustand, gelartig enthalten und so verarbeitbar sind, daß sie sich kostengünstig herstellen lassen.

Erfindungsgemäß wird diese Aufgabe dadurch gelöst, daß die flüssig befüllten Steckkapseln als Wirkstoff Oestradiolvalerat und/oder Medroxyprogesteronacetat zusammen mit einem oder mehreren Verdünnungsmitteln enthalten und die flüssig befüllten Steckkapseln vorzugsweise Hartgelatinesteckkapseln sind.

In einer besonderen Ausführungsform wird zusätzlich ein Verdickungsmittel, vorzugsweise kolloidale Kieselsäure, zur Erhöhung der Konsistenz eingearbeitet.

Steckkapseln im Sinne der neuen technischen Lehre sind solche, die aus einem Unterteil bestehen, welches das Füllgut aufnimmt, und einem Oberteil, welches als Verschluß dient. Steckkapseln der vorgenannten Art werden aus Gelatine, aber auch auf Stärkebasis hergestellt.

Als Verdünnungsmittel eignen sich grundsätzlich alle mit den Steckkapseln und den Wirkstoffen kompatiblen und physiologisch unbedenkliche Hilfsstoffe, wie natürliche Pflanzenöle, Neutralöle, mittelkettige Triglyceride, gesättigte Triglyceride, Polyglykole bzw. Mischungen davon. Bevorzugt verwendet werden Sojaöl, Sesamöl, Miglyol^{(R)} (Hüls-AG, Troisdorf), Softisan^{(R)} (Hüls-AG, Troisdorf) Typ 378,601,649. Darüber hinaus hat sich als weiteres Verdünnungsmittel und Lösungsmittel Dimethylisosorbit als besonders geeignet erwiesen, welches alleine oder zusammen mit einem oder mehreren der vorgenannten Verdünnungsmittel bevorzugt verwendet wird. Sofern die Verdünnungsmittel bei Raumtemperatur eine feste Konsistenz besitzen, erfolgt das Lösen oder die Herstellung der Suspension der Wirkstoffe in der Schmelze.

Flüssige Mischungen aus Wirkstoff und Verdünnungsmittel können als solche oder mit einem Verdicker versetzt werden, mit dem die Mischungen so weit verdickt werden können, daß sie nicht mehr fließfähig sind. Neben Cellulosederivaten wird kolloidale Kieselsäure (Aerosil^{(R)}) bevorzugt verwendet. Darüber hinaus können die erfindungsgemäßen Zusammensetzungen Tenside, Konservierungsmittel, Stabilisatoren sowie Geschmackskorrigenzien enthalten.

Mischungen der vorgenannten Art, insbesondere solche der bevorzugten Zusammensetzung, werden anschließend in Hartgelatinesteckkapseln abgefüllt. Die Abfüllung erfolgt in der Regel bei Raumtemperatur, kann jedoch auch bei höherer Temperatur erfolgen, insbesondere dann, wenn die Wirkstoffe in Schmelzen vorliegen.

Die Steckkapseln können nach dem Befüllen und Verschließen zusätzlich versiegelt oder verklebt werden, um ein Auslaufen sicher auszuschließen. Bevorzugt werden Mischungen der erfindungsgemäßen Art, die so viskos sind, daß sie aus der verschlossenen Kapsel nicht mehr auslaufen können und auf ein zusätzliches Verschließen oder Verkleben der sich überlappenden Kapselteile verzichtet werden kann. Darüber hinaus ist die Umhüllung der Kapsel mit einem Überzug möglich, der den Zerfall der Kapsel erst nach der Magenpassage oder in tieferen Darmabschnitten ermöglicht.

Die erfindungsgemäßen Arzneiformen enthalten in der Regel 0,1 bis 2,5 mg, vorzugsweise 0,5 bis 1,5 mg, Oestradiolvalerat und/oder 0,5 bis 10 mg, vorzugsweise 2,5 bis 7,5 mg, Medroxyprogesteronacetat, wobei die Arzneistoffmenge pro Kapsel je nach der zu verabreichenden Dosis, der Konzentration des Füllgutes und dem Fassungsvermögen der Kapsel variabel sein kann.

Kapseln der vorgenannten Art enthalten den bzw. die Wirkstoffe in hohem Maße gleichverteilt, wobei in den Fällen, in denen der Wirkstoff oder die Wirkstoffkombination gelöst vorliegt, eine gute Resorption ermöglicht wird, dadurch, daß das Lösen der an sich unlöslichen Substanzen nicht mehr den für die Resorption geschwindigkeitsbestimmenden Schritt darstellt.

Die nachfolgenden Beispiele stellen besondere Ausführungsformen der Erfindung dar:

### BEISPIEL 1

0,075 g Oestradiolvalerat
16,000 g Dimethylisosorbit
1,000 g Miglyol
1,682 g kolloidale Kieselsäure
Oestradiolvalerat wird in Dimethylisosorbit bei Raumtemperatur gelöst. Unter Rühren wird nacheinander Miglyol und kolloidale Kieselsäure eingerührt. Es entsteht ein klares, hochviskoses Gel. Das Gel wird in Hartgelatinesteckkapseln der Größe 2 in Mengen von 250 mg abgefüllt, was einer Dosis von 1 mg Oestradiolvalerat pro Kapsel entspricht.

### BEISPIEL 2

0,300 g Medroxyprogesteronacetat
0,075 g Oestradiolvalerat
14,500 g Dimethylisosorbit
2,500 g Miglyol
1,012 g kolloidale Kieselsäure
Oestradiolvalerat und Medroxyprogesteronacetat werden nacheinander in Dimethylisosorbit gelöst. Nacheinander werden Miglyol und die kolloidale Kieselsäure eingerührt. Es entsteht ein klares, hochviskoses Gel, welches in Hartgelatinesteckkapseln der Größe 1 in Mengen von 300 mg, entsprechend 5 mg Medroxyprogesteronacetat und 1,25 mg Oestradiolvalerat pro Kapsel.

### BEISPIEL 3

8,500 g Dimethylisosorbit
0,075 g Oestradiolvalerat
8,500 g Sesamöl
0,793 g kolloidale Kieselsäure
Oestradiolvalerat wird in Dimethylisosorbit gelöst. Nacheinander werden Sesamöl und kolloidale Kieselsäure eingerührt. Es entsteht ein klares, hochviskoses Gel, welches in Hartgelatinesteckkapseln der Größen 4 -000 je nach gewünschter Wirkstoffdosierung abgefüllt wird.

### BEISPIEL 4

17,000 g Sesamöl
0,075 g Oestradiolvalerat
1,065 g kolloidale Kieselsäure
Oestradiolvalerat wird in Sesamöl bei Raumtemperatur gelöst und durch Zugabe von kolloidaler Kieselsäure verdickt. Das Gel wird in Hartgelatinesteckkapseln zu 121 mg abgefüllt, entsprechend 0,5 mg Oestradiolvalerat pro Kapsel.

### BEISPIEL 5

17,000 g Dimethylisosorbit
0,300 g Medroxyprogesteronacetat
1,200 g kolloidale Kieselsäure
Medroxyprogesteronacetat wird in Dimethylisosorbit gelöst und mit kolloidaler Kieselsäure angedickt. Die Mischung wird in Steckkapseln abgefüllt.

## Patentansprüche

1. Flüssig befüllte Steckkapseln, dadurch gekennzeichnet, daß sie als Wirkstoff Oestradiolvalerat und/oder Medroxyprogesteronacetat zusammen mit einem oder mehreren Verdünnungsmitteln enthalten und die flüssig befüllten Steckkapseln verzugsweise Hartgelatinesteckkapseln sind.

2. Flüssig befüllte Steckkapseln nach Anspruch 1, dadurch gekennzeichnet, daß sie zusätzlich ein Verdickungsmittel enthalten.

3. Flüssig befüllte Steckkapseln nach einem der Ansprüche 1 bis 2, dadurch gekennzeichnet, daß als Verdünnungsmittel Dimethylisosorbit oder ein Pflanzenöl oder ein Neutralöl mittelkettiger Triglyceride oder gesättigte Triglyceride oder Mischungen davon enthalten sind.

4. Flüssig befüllte Steckkapseln nach den Ansprüchen 1 bis 3, dadurch gekennzeichnet, daß als Verdickungsmittel kolloidale Kieselsäure enthalten ist.

5. Flüssig befüllte Steckkapseln nach einem der Ansprüche 1 bis 4, dadurch gekennzeichnet, daß sie 0,1 bis 2,5 mg, vorzugsweise 0,5 bis 1,5 mg, Oestradiolvalerat enthalten.

6. Flüssig befüllte Steckkapseln nach einem der Ansprüche 1 bis 5, dadurch gekennzeichnet, daß sie 0,5 bis 10 mg, vorzugsweise 2,5 bis 7,5 mg, Medroxyprogesteronacetat enthalten.

7. Flüssig befüllte Steckkapseln nach einem der Ansprüche 1 bis 6, dadurch gekennzeichnet, daß sie 0,1 bis 2,5 mg, vorzugsweise 0,5 bis 1,5 mg, Oestradiolvalerat und 0,5 bis 10 mg, vorzugsweise 2,5 bis 7,5 mg, Medroxyprogesteronacetat enthalten.

8. Flüssig befüllte Hartgelatinesteckkapseln nach einem der Ansprüche 1 bis 7 zur Verwendung bei der postmenopausalen Hormonsubstitutionstherapie und zur prophylaktischen Verhinderung der Osteoporose.

## Claims

1. Liquid-filled two-piece capsules, characterized in that they contain oestradiol valerate and/or medroxyprogesterone acetate as the active ingredient in combination with one or more diluent(s) and the liquid-filled two-piece capsules preferably are hard gelatin two-piece capsules.

2. Liquid-filled two-piece capsules according to claim 1, characterized in that they additionally contain a thickening agent.

3. Liquid-filled two-piece capsules according to either of claims 1 to 2, characterized in that dimethylisosorbitol or a vegetable oil or a neutral oil of medium-chain triglycerides or saturated triglycerides or mixtures thereof are contained as diluent(s).

4. Liquid-filled two-piece capsules according to anyone of claims 1 to 3, characterized in that colloidal silicic acid is contained as the thickening agent.

5. Liquid-filled two-piece capsules according to anyone of claims 1 to 4, characterized in that they contain from 0.1 to 2.5 mg, and preferably from 0.5 to 1.5 mg, of oestradiol valerate.

6. Liquid-filled two-piece capsules according to anyone of claims 1 to 5, characterized in that they contain from 0.5 to 10 mg, and preferably from 2.5 to 7.5 mg, of medroxyprogesterone acetate.

7. Liquid-filled two-piece capsules according to anyone of claims 1 to 6, characterized in that they contain from 0.1 to 2.5 mg, and preferably from 0.5 to 1.5 mg, of oestradiol valerate and from 0.5 to 10 mg, and preferably from 2.5 to 7.5 mg, of medroxyprogesterone acetate.

8. Liquid-filled two-piece hard gelatin capsules according to anyone of claims 1 to 7 for use in postmenopausal hormone substitution therapy and for prophylactic prevention of osteoporosis.

## Revendications

1. Des ovules remplis de liquide, caractérisés en ce qu'ils contiennent comme principe actif du valérate d'oestradiol et/ou de l'acétate de médroxyprogestérone avec un ou plusieurs diluants, et que les ovules remplis de liquide sont de préférence des ovules en gélatine dure.

2. Des ovules remplis de liquide selon la revendication 1, caractérisés en ce qu'ils contiennent en outre un agent épaisissant.

3. Des ovules remplis de liquide selon une des revendications 1 ou 2, caractérisés en ce qu'ils contiennent comme diluant de l'isosorbite de diméthyle ou une huile végétale ou une huile neutre de triglycéride à chaîne de taille moyenne ou de triglycéride saturé ou des mélanges de ces composants.

4. Des ovules remplis de liquide selon les revendications 1 à 3, caractérisés en ce qu'ils contiennent comme agent épaississant de l'acide silicique colloïdal.

5. Des ovules remplis de liquide selon une des revendications 1 à 4, caractérisés en ce qu'ils contiennent 0,1 à 2,5 mg. - de préférence 0,5 à 1,5 mg. - de valérate d'oestradiol.

6. Des ovules remplis de liquide selon une des revendications 1 à 5, caractérisés en ce qu'ils contiennent 0,5 à 10 mg. - de préférence 2,5 à 7,5 mg. - d'acétate de médroxyprogestérone.

7. Des ovules remplis de liquide selon une des revendications 1 à 6, caractérisés en ce qu'ils contiennent 0,1 à 2,5 mg. - de préférence 0,5 à 1,5 mg. - de valérate d'oestradiol et 0,5 à 10 mg. - de préférence 2,5 à 7,5 mg.-d'acétate de médroxyprogestérone.

8. Des ovules en gélatine dure remplis de liquide selon une des revendications 1 à 7 pour être utilisés dans la thérapie postménopausique de substitution hormonale et dans le traitement prophylactique d'ostéoporose.
